# EUROPÄISCHE PATENTSCHRIFT

(11) **EP 0 852 580 B1**
(45) Veröffentlichungstag und Bekanntmachung des Hinweises auf die Patenterteilung: **25.10.2000**
(21) Anmeldenummer: 96930988.9
(22) Anmeldetag: 30.08.1996
(51) Int. Cl.: C07D 239/54, C12P 7/62, C12P 13/02

(54) **VERFAHREN ZUR HERSTELLUNG VON DIHYDROXYPYRIMIDIN-DERIVATEN**
METHOD OF PRODUCING DIHYDROXYPYRIMIDINE DERIVATIVES
PROCEDE DE PREPARATION DE DERIVES DE DIHYDROXYPYRIMIDINE

(30) Priorität: 31.08.1995 CH 247495
(43) Veröffentlichungstag der Anmeldung: 15.07.1998
(73) Patentinhaber: LONZA AG, CH-3945 Gampel/Wallis (CH)
(72) Erfinder: SCHMIDT, Beat, CH-3937 Baltschieder (CH); KIENER, Andreas, CH-3930 Visp (CH); McGARRITY, John, CH-3902 Glis (CH)
(74) Vertreter: Weinhold, Peter, Dr.rer.nat. Dipl.-Chem.
(86) Internationale Anmeldenummer: EP9603826
(87) Internationale Veröffentlichungsnummer: WO9708152

(56) Entgegenhaltungen:
- FR-A- 1 424 940
- JOURNAL OF THE CHEMICAL SOCIETY, Nr. 448, 1956, LETCHWORTH GB, Seiten 2313-2314, XP002020558 D.J.BROWN: "PYRIMIDINE REACTIONS.PART 1,FROM MALONDIAMIDE." in der Anmeldung erwähnt
- CHEMICAL ABSTRACTS, vol. 122, no. 3, 1995 Columbus, Ohio, US; abstract no. 29899y, Seite 794; XP002020559 & JP,A,06 256 278 (NISSAN CHEMICAL IND.) 13.September 1994
- CHEMICAL ABSTRACTS, vol. 123, no. 3, 1995 Columbus, Ohio, US; abstract no. 31412y, Seite 704; XP002020560 & JP,A,00 799 983 (NIPPON KAYAKU) 18.April 1995

## Beschreibung

Die Erfindung betrifft ein neues Verfahren zur Herstellung von Dihydroxypyrimidin-Derivaten der allgemeinen Formel 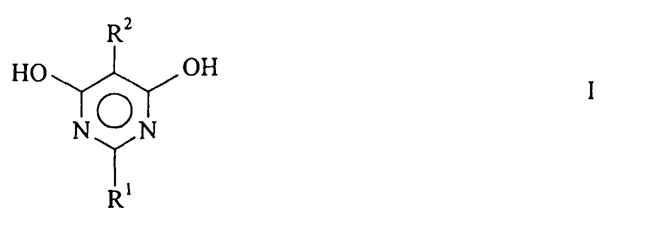 worin R¹ und R² gleich oder verschieden sind und ein Wasserstoffatom, Aryl- oder eine C₁-C₄-Alkylgruppe bedeuten, ausgehend von einer Verbindung der allgemeinen Formel 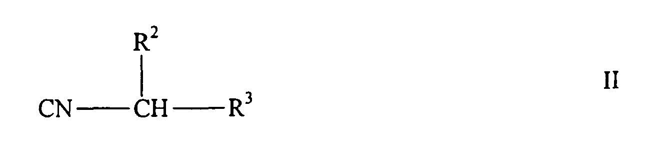 worin R² die genannte Bedeutung hat und R³ ―CN oder COOR⁴ bedeutet, worin R⁴ eine C₁-C₄-Alkylgruppe ist.

Dihydroxypyrimidin ist ein wichtiges Zwischenprodukt zur Herstellung von Insektiziden wie z. B. zur Herstellung von 4,6-Pyrimidin-diyl-bis-(thiono)(thiol)-phosphor(phosphon)-säurestern (DE 25 23 324).

Bisher sind mehrere Verfahren zur Herstellung von Dihydroxypyrimidin bzw. deren Derivaten bekannt.

Bekannt ist beispielsweise die Herstellung von Dihydroxypyrimidin, ausgehend von Malonsäurediamid (DE-PS 12 00 308). Dabei wird Malonsäurediamid mit Formamid in Gegenwart von Natriumethanolat zum Dihydroxypyrimidin cyclisiert. Dieses Verfahren hat den Nachteil, dass das Edukt Malonsäurediamid realtiv kostspielig ist.

D. J. Brown (J. Chem. Soc., 1956, S. 2312 - 2314) beschreibt ebenfalls ein Verfahren zur Herstellung von Dihydroxypyrimidin, ausgehend von Malonsäurediamid. Dabei wird Malonsäurediamid in Gegenwart von Natriumethoxid und Ethylformiat zum Dihydroxypyrimidin cyclisiert. Dieses Verfahren hat zum einen den Nachteil, dass Dihydroxypyrimidin nur in mässiger Ausbeute erhalten wird. Zum anderen ist, wie bereits oben beschrieben, das Edukt Malonsäurediamid relativ kostspielig.

Die JP-4260 umfasst ein Verfahren zur Herstellung von Dihydroxypyrimidin durch Reaktion von Malonat mit Formamid in Gegenwart eines Alkalimetallalkoxids. Nachteilig bei diesem Verfahren ist, dass das Formamid in grossem Überschuss verwendet werden muss.

Die US-PS 17 66 748 beschreibt ein Verfahren zur Herstellung von 2-Aryl-4,6-dihydroxypyrimidinen, ausgehend von Malonsäurediethylester. Dabei wird Malonsäurediethylester in Gegenwart eines Amidins einer Arylcarbonsäure zum entsprechenden Produkt cyclisiert. Dieses Verfahren hat den Nachteil, dass die entsprechenden Amidine sehr kostspielig sind.

Aufgabe der vorliegenden Erfindung war, ein wirtschaftlicheres und ökologisch günstigeres Verfahren zur Herstellung von Dihydroxypyrimidinderivaten zur Verfügung zu stellen, wobei Dihydroxypyrimidin in guter Ausbeute und Reinheit isoliert werden kann.

Diese Aufgabe wurde mit dem erfindungsgemässen Verfahren gemäss Anspruch 1 gelöst.

In der ersten Verfahrensstufe wird als Substrat eine Verbindung der allgemeinen Formel 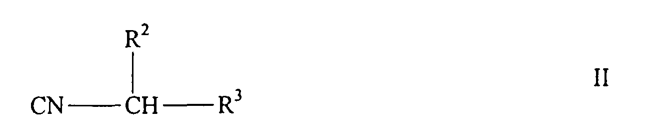 worin R² und R³ die genannte Bedeutung haben, mittels Mikroorganismen der Gattung *Rhodococcus* in ein Malonsäurederivat der allgemeinen Formel 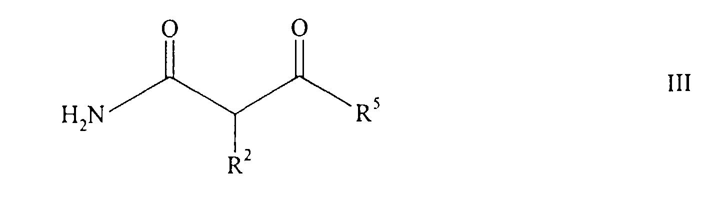 worin R² die genannte Bedeutung hat und R⁵ eine C₁-C₄-Alkoxygruppe oder NH₂ bedeutet, umgesetzt.

Die Verbindungen der allgemeinen Formel II wie Cyanessigsäuremethyl- oder -ethylester sind käufliche Verbindungen.

Zweckmässig wird die erste Stufe mit Mikroorganismen der Spezies *Rhodococcus rhodochrous, Rhodococcus sp.* 5 - 6 oder *Rhodococcus equi*, vorzugsweise mit Mikroorganismen der Spezies *Rhodococcus sp.* 5 - 6 (FERM BP-687), *Rhodococcus rhodochrous* J1 (FERM BP-1478) oder mit Mikroorganismen der Spezies *Rhodococcus equi* TG328 (FERM BP-3791 bzw. DSM 6710), durchgeführt. Insbesondere wird die Umsetzung mittels Mikroorganismen der Spezies *Rhodococcus rhodochrous* (FERM BP- 1478) durchgeführt. Die Mikroorganismen der Spezies *Rhodococcus sp.* 5 - 6, *Rhodococcus rhodochrous* J1 und *Rhodococcus equi* TG328 sind in der Literatur beschriebene Mikroorganismen. *Rhodococcus rhodochrous* J1 (FERM BP-1478) ist in der EP-B 307 928, *Rhodococcus sp.* 5 - 6 (FERM BP-687) in der EP-A 0 188 316 und *Rhodococcus equi* TG328 (FERM BP-3791) in der US-PS 5 258 305 ausführlich beschrieben.

Für das Verfahren ebenfalls geeignet sind die funktionell aequivalenten Varianten und Mutanten dieser Mikroorganismen. Unter "funktionell aequivalenten Varianten und Mutanten" werden Mikroorganismen verstanden, die im wesentlichen dieselben Eigenschaften und Funktionen wie die Ursprungsmikroorganismen besitzen. Derartige Varianten und Mutanten können zufällig z. B. durch UV-Bestrahlung gebildet werden.

Üblicherweise werden die Mikroorganismen vor der eigentlichen Biotransformation entsprechend der EP-B 307 928 kultiviert (angezüchtet) und die wirksamen Enzyme induziert. Vorzugsweise erfolgt die Biotransformation mit, auf fachmännisch übliche Weise, immobilisierten Mikroorganismen-Zellen.

Zweckmässig wird die Biotransformation in einem pH-Bereich von 3 bis 7 vorzugsweise in einem pH-Bereich von 4 bis 6 durchgeführt.

Die Biotransformation kann bei einer Temperatur von 0 bis 30 °C, vorzugsweise von 3 bis 20 °C durchgeführt werden.

Als Substrate werden Verbindungen der allgemeinen Formel II verwendet, worin R² ein Wasserstoffatom, Aryl- oder eine C₁-C₄-Alkylgruppe und R³ ―CN oder COOR⁴ bedeutet, worin R⁴ eine C₁-C₄-Alkylgruppe ist. Als C₁-C₄-Alkylgruppe kann Methyl-, Ethyl-, Propyl-, i-Propyl-, Butyl-, i-Butyl- oder t-Butyl-, verwendet werden. Als Aryl- kann beispielsweise Phenyl-, substituiert oder unsubstituiert, oder Naphtyl- verwendet werden. Vorzugsweise bedeutet R² ein Wasserstoffatom und R³ Methyl-, Ethyl-, i-Propyl- oder ―CN.

Nach einer üblichen Umsetzungszeit von 1 bis 100 h können die gebildeten Amide auf einfache Weise z. B. durch Abtrennen von Wasser isoliert werden.

In der zweiten Verfahrensstufe wird das Malonsäurederivat der allgemeinen Formel 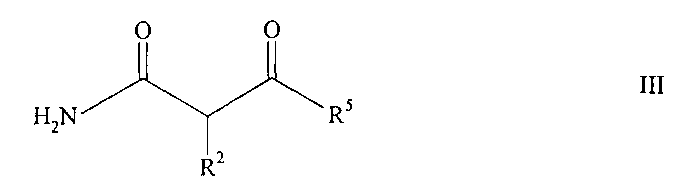 mit einem Carbonsäureamid der allgemeinen Formel 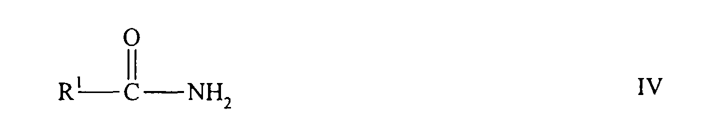 in Gegenwart einer Base zum Endprodukt gemäss Formel I cyclisiert.

Rest R⁵ bedeutet eine C₁-C₄-Alkoxygruppe wie Methoxy-, Ethoxy-, Propoxy-, Butoxy-, i-Butoxy-, t-Butoxy- oder ―NH₂. Vorzugsweise bedeutet R⁵ Methoxy- oder Ethoxy-. Rest R² hat die bereits beschriebene Definition.

Rest R¹ bedeutet entweder C₁-C₄-Alkyl- wie Methyl-, Ethyl-, Propyl-, i-Propyl-, Butyl-, t-Butyl-, i-Butyl- oder ein Wasserstoffatom. Vorzugsweise bedeutet R¹ ein Wasserstoffatom.

Zweckmässig wird das Carbonsäureamid in einem Verhältnis von 2 bis 8 mol pro mol Malonsäurederivat, vorzugsweise in einem Verhältnis von 2 bis 3 mol verwendet.

Als Base wird zweckmässig ein Alkalimetallalkoholat wie Natrium- oder Kalium-methanolat, -ethanolat, -propanolat, -butanolat, i-butanolat, t-butanolat, amylat- oder i-amylat verwendet. Vorzugsweise wird Natriummethanolat verwendet.

Die Konzentration der Base kann in einem Bereich von 2 bis 6 mol pro mol Malonsäurederivat, vorzugsweise in einem Bereich von 3 bis 4 mol variieren.

Als Lösungsmittel können für die zweite Stufe polare Lösungsmittel wie Methanol, Ethanol, Propanol oder Butanol verwendet werden, vorzugsweise wird Methanol verwendet.

Zweckmässig wird die zweite Stufe bei einer Temperatur von 30 °C bis zur Rückflusstemperatur des entsprechenden Lösungsmittels, vorzugsweise bei Rückflusstemperatur des entsprechenden Lösungsmittels, durchgeführt.

Nach einer weiteren Umsetzungszeit von 1 bis 6 h kann das Dihydroxypyrimidinderivat der Formel I durch übliche Aufarbeitungsmethoden isoliert werden.

### Beispiele

### Beispiel 1

### Herstellung von Malonsäure-monoamid-monoester (Carbamoyl-essigsäure-ester) aus Cyanessigsäuremethylester, Cyanessigsäureethylester und Cyanessigsäureisoproylester

In ein Glasgefäss, welches mit einem Magnetrührer ausgerüstet war, wurden 200 ml Wasser, 0,62 g J1 Biomasse (FERM BP-1478) (berechnet als Trockengewicht) und 50 g des entsprechenden Cyanessigsäure-esters bei Raumtemperatur während 16 Std inkubiert. Der pH-Wert lag etwa bei 5. Mittels GC-Analytik konnte zu diesem Zeitpunkt kein Ausgangsmaterial mehr nachgewiesen werden. Die Biomasse wurde abfiltriert und das Wasser vom Produkt unter reduziertem Druck entfernt. Spuren von Wasser wurden danach durch Zugabe von Toluol zum Rückstand azeotrop entfernt. Die isolierte Ausbeute betrug >90% und die Reinheit der gebildeten Carbamoyl-essigsäure-ester betrug nach GC >95%. Carbamoyl-essigsäure-isopropylester präzipitierte unter diesen Bedingungen während der Biotransformation aus wässeriger Lösung. In diesem Falle wurde die Suspension zuerst auf 50 °C erwärmt, um den Isopropylester in Lösung zu bringen, bevor die Biomasse abfiltriert wurde.

### Beispiel 2

### Einfluss des pH-Wertes auf die Umsetzung von Cyanessigsäuremethylester zu Carbamoylessigsäuremethylester.

Für die Versuche wurde ein 1 l Applikon Fermenter verwendet. Der Biotransformationsansatz enthielt 200 g Cyanessigsäuremethylester, 800 ml Wasser und 1,2 g J1 Biomasse (FERM BP-687) (berechnet als Trockengewicht). Das Gefäss wurde mit 200 UpM gerührt und die Temperatur betrug 12 °C - 17 °C. Die Umsetzung wurde nach 16 h abgebrochen. Zu diesem Zeitpunkt konnte mittels GC-Analytik kein Ausgangsmaterial nachgewiesen werden. Der pH-Wert wurde im Ansatz A nicht konstant gehalten und fiel von einem Anfangswert von 5,7 auf 4,7 am Ende der Reaktion.
Im Ansatz B wurde der pH während der gesamten Reaktionszeit auf einen Wert von 8,0 konstant gehalten. Dazu wurde 30%-NaOH-Lösung verwendet.
Die Aufarbeitung erfolgte wie oben beschrieben.

Mittels Titration konnte sowohl in Ansatz A als auch in Ansatz B eine signifikante Menge an Carbamoylessigsäuremethylester nachgewiesen werden. Im Gegensatz zu Ansatz B war jedoch das Produkt in Ansatz A wesentlich reiner.

### Beispiel 3

### Umsetzung von Malonsäuredinitril zu Malonsäurediamid mit J1 Biomasse (FERM BP-1478)

In ein Glasgefäss, welches mit einem Magnetrührer ausgerüstet war, wurde 90 ml Wasser, 0,3 g J1 Biomasse (berechnet als Trockengewicht) und 10 g Malonsäuredinitril bei Raumtemperatur während 6 h inkubiert. Mittels GC-Analytik konnte zu diesem Zeitpunkt kein Ausgangsmaterial mehr nachgewiesen werden. Malonsäurediamid präzipitierte unter diesen Bedingungen während der Biotransformation aus wässeriger Lösung. Die Suspension wurde auf 50 °C erwärmt, um das Produkt in Lösung zu bringen, bevor die Biomasse abfiltriert wurde.
Das Wasser wurde vom Produkt unter reduziertem Druck entfernt. Die isolierte Ausbeute betrug >90% und die Reinheit des gebildeten Malonsäureadiamide betrug >95% (GC).

### Beispiel 4

### Herstellung von Dihydroxypyrimidin (R¹ = R² = H)

Formamid (26,43 g; 575 mmol) wurde zu Natriummethylat Lösung (157,56 g; 875 mmol) bei Raumtemperatur während 5 Minuten zugefügt, wobei sich die Lösung auf 28 °C erwärmte. Die Lösung wurde auf Rückfluss (64 °C) erhitzt und 15 Minuten bei dieser Temperatur gehalten. Zu dieser 64 °C warmen Lösung tropfte man während 60 min Malonsäuremonoamidmonoester, in 30 ml Methanol gelöst, zu. Dabei entstand allmählich eine farblose Suspension. Man kochte die leicht rührbare Suspension 3 h unter Rückfluss und kühlte anschliessend auf Raumtemperatur ab. Man fügte 125 ml Wasser so zu, dass die Temperatur ca 25 °C betrug. Am Ende der Zugabe erhielt man eine leicht gelbe Lösung. Man stellte den pH auf 4,0 mit der Zugabe von 82,0 g konz. HCl-Lösung (die Temperatur lag zwischen 25 und 30 °C) ein. Die Suspension liess man 15 min bei Raumtemperatur rühren, filtrierte den ausgefallenen Feststoff ab und wusch 2mal mit 45 ml Wasser tüchtig nach. Der hellgelbe Feststoff wurde im Vakuumtrockenschrank bei 60 °C während 24 h getrocknet. Es wurden 22,67 g (Gehalt 97,0% nach HPLC), entsprechend einer Ausbeute von 78,5% erhalten.

| | |
|---|---|
| ¹H NMR (DMSO_{*d6*}) δ | 5,22 (s, 1H); |
| | 8,05 (s, 1H); |
| | 11,5 - 12,2 (s, *br*, 2H). |
| | |
| ¹³C NMR (DMSO_{*d6*}) δ | 89,99; |
| | 149,90; |
| | 166,17. |

## Patentansprüche

1. Verfahren zur Herstellung von Dihydroxypyrimidin-Derivaten der allgemeinen Formel 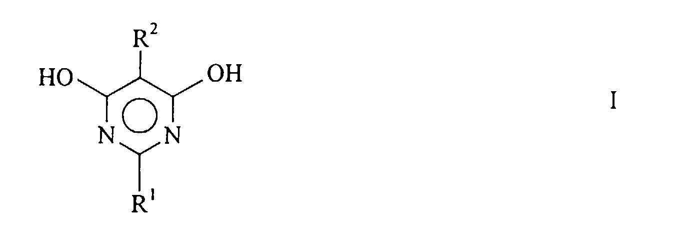 worin R¹ und R² gleich oder verschieden sind und ein Wasserstoffatom, eine C₁-C₄-Alkylgruppe oder eine Arylgruppe bedeuten, dadurch gekennzeichnet, dass man in der ersten Stufe als Substrat eine Verbindung der allgemeinen Formel 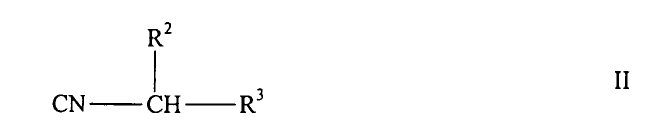 worin R² die genannte Bedeutung hat und R³ ―CN oder COOR⁴ bedeutet, worin R⁴ eine C₁-C₄-Alkylgruppe ist, mittels Mikroorganismen der Gattung *Rhodococcus* in ein Malonsäurederivat der allgemeinen Formel 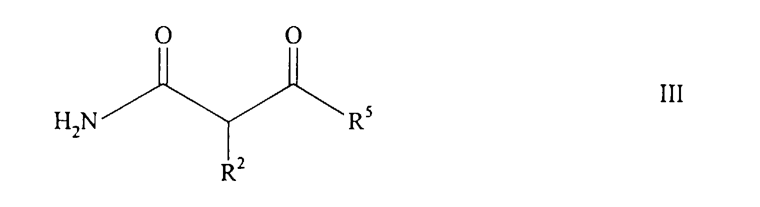 worin R² die genannte Bedeutung hat und R⁵ eine C₁-C₄-Alkoxygruppe oder ―NH₂ bedeutet, umsetzt und dieses in der zweiten Stufe mit einem Carbonsäureamid der allgemeinen Formel 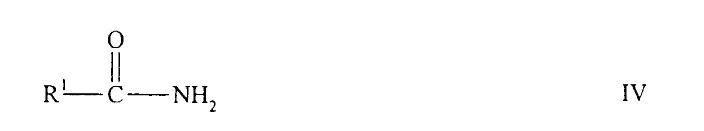 worin R¹ die genannte Bedeutung hat in Gegenwart einer Base zum Endprodukt gemäss Formel I cyclisiert.

2. Verfahren nach Patentanspruch 1, dadurch gekennzeichnet, dass man die Umsetzung in der ersten Stufe mittels Mikroorganismen der Spezies *Rhodococcus rhodochrous* oder mit deren funktionell aequivalenten Varianten und Mutanten durchführt.

3. Verfahren nach Patentanspruch 2, dadurch gekennzeichnet, dass man die Umsetzung in der ersten Stufe mittels immobilisierten Mikroorganismen der Spezies *Rhodococcus rhodochrous* oder mit deren funktionell aequivalenten Varianten und Mutanten durchführt.

4. Verfahren nach mindestens einem der Patentansprüche 1 bis 3, dadurch gekennzeichnet, dass man die Umsetzung in der ersten Stufe bei einem pH von 3 bis 7 und einer Temperatur von 0 bis 30 °C durchführt.

5. Verfahren nach mindestens einem der Patentansprüche 1 bis 4, dadurch gekennzeichnet, dass man in der ersten Stufe als Verbindung der allgemeinen Formel II Cyanessigsäuremethyl-, Cyanessigsäureethyl-, Cyanessigsäureisopropylester oder Malonsäuredinitril verwendet.

6. Verfahren nach mindestens einem der Patentansprüche 1 bis 5, dadurch gekennzeichnet, dass man in der zweiten Stufe als Malonsäurederivat Malonsäuremonoamidmonomethyl- oder Malonsäuremonoamidmonoethylester verwendet.

7. Verfahren nach mindestens einem der Patentansprüche 1 bis 6, dadurch gekennzeichnet, dass man in der zweiten Stufe als Carbonsäureamid Formamid verwendet.

8. Verfahren nach mindestens einem der Patentansprüche 1 bis 7, dadurch gekennzeichnet, dass man in der zweiten Stufe als Base ein Alkalimetallalkoholat verwendet.

9. Verfahren nach mindestens einem der Patentansprüche 1 bis 8, dadurch gekennzeichnet, dass man die zweite Stufe bei einer Temperatur von 30 °C bis zur Rückflusstemperatur des entsprechenden Lösungsmittels durchführt.

10. Verfahren zur Herstellung von Malonsäurederivaten der allgemeinen Formel 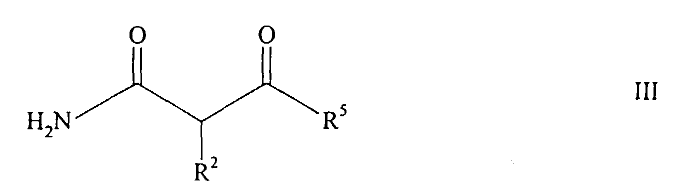 worin R² und R⁵ die in Anspruch 1 genannte Bedeutung haben, dadurch gekennzeichnet, dass man eine Verbindung der allgemeinen Formel 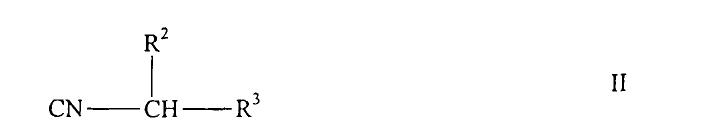 worin R² und R³ die in Anspruch 1 genannte Bedeutung haben, mittels Mikroorganismen der Gattung *Rhodococcus* in das Malonsäurederivat der allgemeinen Formel III überführt.

11. Verfahren nach Patentanspruch 10, dadurch gekennzeichnet, dass man die Umsetzung bei einer Temperatur von 0 bis 30 °C und bei einem pH von 3 bis 7 durchführt.

## Claims

1. Method of producing dihydroxypyrimidine derivatives of the general formula 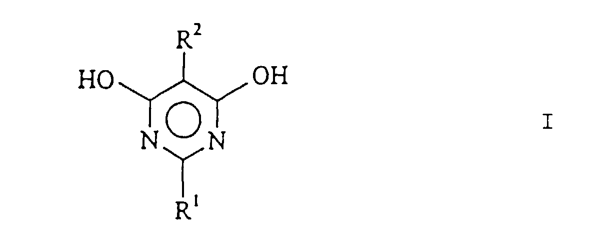 in which R¹ and R² are the same or different and mean a hydrogen atom, a C₁-C₄-alkyl group or an aryl group, characterized in that in the first stage as substrate a compound of the general formula 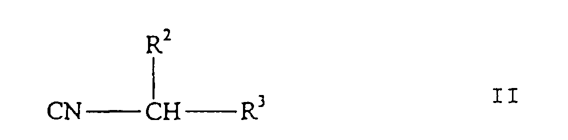 in which R² has the said meaning and R³ means -CN or COOR⁴, in which R⁴ is a C₁-C₄-alkyl group, is converted by means of microorganisms of the genus *Rhodococcus* into a malonic acid derivative of the general formula 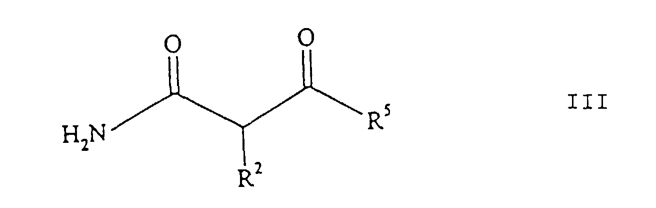 in which R² has the said meaning and R⁵ means a C₁-C₄-alkoxy group or -NH₂ and in the second stage the malonic acid derivative is cyclized to the end product according to Formula I in the presence of a base, with a carboxylic acid amide of the general formula 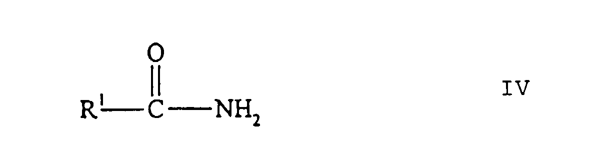 in which R¹ has the said meaning.

2. Method according to Claim 1, characterized in that the conversion in the first stage is carried out by means of microorganisms of the species *Rhodococcus rhodochrous* or with functionally equivalent variants and mutants thereof.

3. Method according to Claim 2, characterized in that the conversion in the first stage is carried out by means of immobilized microorganisms of the species *Rhodococcus rhodochrous* or with functionally equivalent variants and mutants thereof.

4. Method according to at least one of Claims 1 to 3, characterized in that the conversion in the first stage is carried out at a pH of 3 to 7 and a temperature of 0 to 30 °C.

5. Method according to at least one of Claims 1 to 4, characterized in that in the first stage cyanoacetic acid methyl-, cyanoacetic acid ethyl-, cyanoacetic acid isopropyl ester or malonitrile is used as compound of the general formula II.

6. Method according to at least one of Claims 1 to 5, characterized in that in the second stage malonic acid monoamide monomethyl- or malonic acid monoamide monoethylester is used as malonic acid derivative.

7. Method according to at least one of Claims 1 to 6, characterized in that in the second stage formamide is used as carboxylic acid amide.

8. Method according to at least one of Claims 1 to 7, characterized in that in the second stage an alkali metal alcoholate is used as base.

9. Method according to at least one of Claims 1 to 8, characterized in that the second stage is carried out at a temperature of 30 °C up to the reflux temperature of the corresponding solvent.

10. Method of producing malonic acid derivatives of the general formula 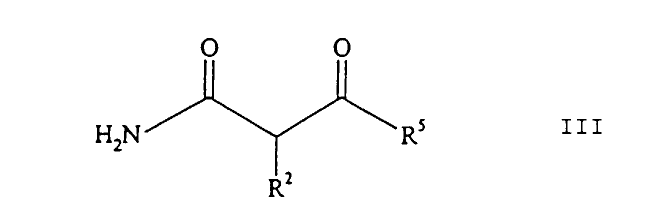 in which R² and R⁵ have the meaning stated in Claim 1, characterized in that a compound of the general formula 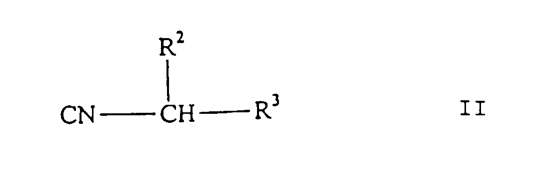 in which R² and R³ have the meaning stated in Claim 1, is transferred into the malonic acid derivative of the general formula III by means of microorganisms of the genus *Rhodococcus.*

11. Method according to Claim 10, characterized in that the conversion is carried out at a temperature of 0 to 30 °C and a pH of 3 to 7.

## Revendications

1. Procédé de préparation de dérivés de dihydroxypyrimidine de formule générale 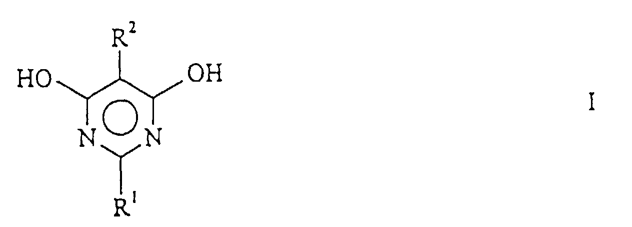 dans laquelle R¹ et R² sont identiques ou différents et représentent un atome d'hydrogène, un groupe alkyle en C₁-C₄ ou un groupe aryle, caractérisé en ce que, dans une première étape, on transforme à l'aide de micro-organismes du genre *Rhodococcus* un composé de formule générale 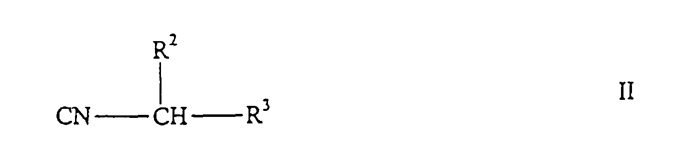 dans laquelle R² a la signification indiquée et R³ représente un groupe CN ou COOR⁴, où R⁴ est un groupe alkyle en C₁-C₄, comme substrat, en un dérivé d'acide malonique de formule générale 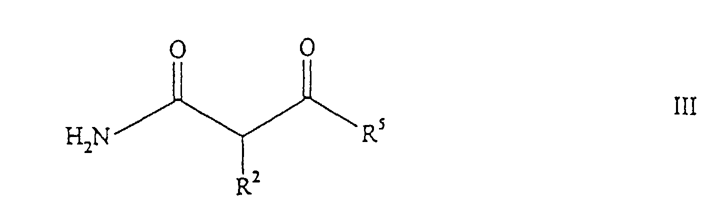 dans laquelle R² a la signification indiquée et R⁵ représente un groupe alcoxy en C₁-C₄ ou NH₂, et on cyclise celui-ci dans une deuxième étape avec un amide d'acide carboxylique de formule générale 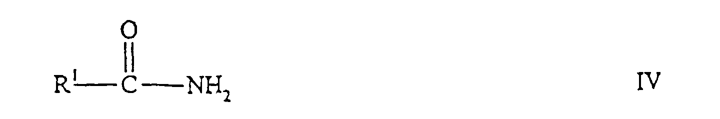 dans laquelle R¹ a la signification indiquée, en présence d'une base, pour obtenir le produit final de formule I.

2. Procédé selon la revendication 1, caractérisé en ce que, dans la première étape, on effectue la transformation à l'aide de micro-organismes de l'espèce *Rhodococcus rhodochrous* ou avec leurs variantes et mutants fonctionnellement équivalents.

3. Procédé selon la revendication 2, caractérisé en ce que, dans la première étape, on effectue la transformation à l'aide de micro-organismes immobilisés de l'espèce *Rhodococcus rhodochrous* ou avec leurs variantes et mutants fonctionnellement équivalents.

4. Procédé selon l'une au moins des revendications 1 à 3, caractérisé en ce que, dans la première étape, on effectue la transformation à un pH de 3 à 7 et à une température de 0 à 30°C.

5. Procédé selon l'une au moins des revendications 1 à 4, caractérisé en ce que, dans la première étape, on utilise comme composé de formule générale II du cyanoacétate de méthyle, d'éthyle ou d'isopropyle ou le dinitrile de l'acide malonique.

6. Procédé selon l'une au moins des revendications 1 à 5, caractérisé en ce que, dans la deuxième étape, on utilise comme dérivé de l'acide malonique l'ester monométhylique du monoamide de l'acide malonique ou l'ester monoéthylique du monoamide de l'acide malonique.

7. Procédé selon l'une au moins des revendications 1 à 6, caractérisé en ce que, dans la deuxième étape, on utilise le formamide comme amide d'acide carboxylique.

8. Procédé selon l'une au moins des revendications 1 à 7, caractérisé en ce que, dans la deuxième étape, on utilise comme base un alkylate de métal alcalin.

9. Procédé selon l'une au moins des revendications 1 à 8, caractérisé en ce que l'on effectue la deuxième étape à une température de 30°C à la température de reflux du solvant correspondant.

10. Procédé de préparation de dérivés de l'acide malonique de formule générale III 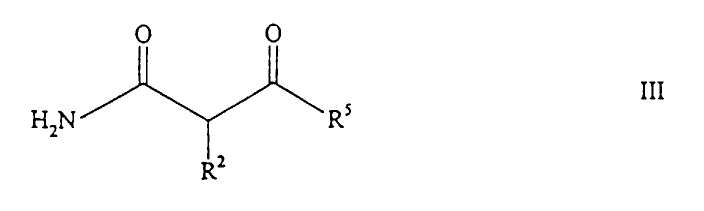 dans laquelle R² et R⁵ ont la signification indiquée dans la revendication 1, caractérisé en ce que l'on transforme à l'aide de micro-organismes du genre *Rhodococcus* un composé de formule générale 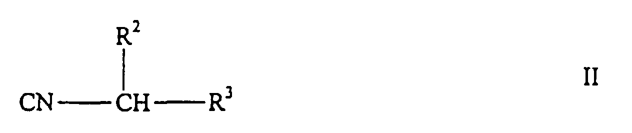 dans laquelle R² et R³ ont la signification indiquée dans la revendication 1, en le dérivé d'acide malonique de formule générale III.

11. Procédé selon la revendication 10, caractérisé en ce que l'on effectue la réaction à une température de 0 à 30°C et à un pH de 3 à 7.
